# EUROPÄISCHE PATENTSCHRIFT

(11) **EP 3 636 289 B1**
(45) Veröffentlichungstag und Bekanntmachung des Hinweises auf die Patenterteilung: **29.09.2021**
(21) Anmeldenummer: 18199678.6
(22) Anmeldetag: 10.10.2018
(51) Int. Cl.: A61L 27/04, A61L 27/50, A61L 31/02, A61L 31/18

(54) **RESORBIERBARES IMPLANTATMATERIAL AUS MAGNESIUM ODER EINER MAGNESIUMLEGIERUNG MIT DOTIERTEN NANODIAMANTEN**
RESORBABLE IMPLANT MATERIAL MADE OF MAGNESIUM OR A MAGNESIUM ALLOY WITH DOPED NANODIAMONDS
MATÉRIAU D'IMPLANT RÉSORBABLE DE MAGNÉSIUM OU D'UN ALLIAGE DE MAGNÉSIUM À NANODIAMANTS DOPÉS

(43) Veröffentlichungstag der Anmeldung: 15.04.2020
(73) Patentinhaber: Helmholtz-Zentrum hereon GmbH, 21502 Geesthacht (DE)
(72) Erfinder: Dieringa, Hajo, 21335 Lüneburg (DE)
(74) Vertreter: Uexküll & Stolberg

(56) Entgegenhaltungen:
- WO-A2-2013/158869
- BO-RONG LIN ET AL: "Fe Doped Magnetic Nanodiamonds Made by Ion Implantation as Contrast Agent for MRI", SCIENTIFIC REPORTS, Bd. 8, Nr. 1, 4. Mai 2018 (2018-05-04), XP055578273, DOI: 10.1038/s41598-018-25380-1

## Beschreibung

### GEBIET DER ERFINDUNG

Die vorliegende Erfindung betrifft ein Implantatmaterial aus Magnesium oder einer Magnesiumlegierung und ein Verfahren zur Herstellung desselben.

### HINTERGRUND DER ERFINDUNG

Sowohl in der Veterinär- als auch in der Humanmedizin werden derzeit zur Versorgung von Frakturen gewichtstragender langer Röhrenknochen Metallimplantate aus medizinischem Stahl oder Titan verwendet. Diese Implantate sind in ihrem mechanischen Verhalten jedoch rigider als Knochen, was zum Phänomen der Belastungsabschirmung ("stress-shielding") führen kann. Aus diesen und anderen Gründen werden entsprechende Implantate in der Regel nach Funktionserfüllung wieder entfernt, was für den Patienten durch die notwendige Narkose und das erneute Gewebetrauma belastend sein kann.

Resorbierbare Implantate werden zunehmend zur Frakturversorgung interessant. Ziel ist es, dass die Implantate mit zunehmender Festigkeit des heilenden Knochens eine Belastungsanpassung durch langsame Abnahme ihrer Stabilität erfahren. Der Einsatz der bisher zur Verfügung stehenden resorbierbaren Implantate aus unterschiedlichen Polymeren gelingt aufgrund ihrer niedrigen Festigkeiten am belasteten Knochen nicht optimal. Dagegen weisen Magnesium und seine Legierungen im Vergleich zu anderen metallischen Implantatmaterialien ein den Knochen ähnliches Elastizitätsmodul und günstige Zug- und Druckfestigkeit auf. Magnesium und seine Legierungen besitzen höhere Festigkeiten und einen größeren Elastizitätsmodul als resorbierbare Polymere und stehen daher im Fokus der wissenschaftlichen Forschung. Bioresorbierbare Implantate, insbesondere aus Magnesium oder einer Magnesiumlegierung zur Behandlung von Knochenfrakturen sind beispielsweise aus der EP 2 318 057 B1 und darin zitierten Druckschriften oder der DE 10 2005 060 203 A1 bekannt.

Resorbierbare Implantate werden nicht nur zur Frakturversorgung eingesetzt. Heutzutage werden Implantate aus Magnesium und seinen Legierungen besonders häufig als Stents eingesetzt, die zur Behandlung von Stenosen (Gefäßverengungen) dienen. Stents weisen ein rohrförmiges oder hohlzylinderförmiges Grundgitter auf, dass an beiden Längsenden offen ist. Das rohrförmige Grundgitter einer derartigen Endoprothese wird in das zu behandelnde Gefäß eingesetzt und dient dazu, das Gefäß zu stützen. Biodegradable Stents aus Magnesium oder Magnesiumlegierung sind beispielsweise aus der EP 2 198 898 B1 und darin zitierten Druckschriften bekannt.

WO 2013/158869 A offenbart ein Implantat aus Magnesium mit eingelagerten Nanodiamanten.

Nachteil der bekannten Implantate ist jedoch, dass die Lage des Implantats während des medizinischen Eingriffs zu ihrer Implantierung bzw. unmittelbar danach nur mittels Röntgenuntersuchungen ermittelt werden konnte. Auch die Resorption des Implantats kann bisher nur mittels Röntgenuntersuchungen verfolgt werden. Diese Untersuchungen sind vergleichsweise aufwendig und kostenintensiv.

Aufgabe der vorliegenden Erfindung ist es, ein Implantatmaterial aus Magnesium oder einer Magnesiumlegierung und ein Verfahren zur Herstellung desselben zur Verfügung zu stellen, dessen Lage während des medizinischen Eingriffs zu seiner Implantierung und dessen Resorption im Körper des Patienten auf einfache Weise verfolgt werden kann.

### ZUSAMMENFASSUNG DER ERFINDUNG

Gelöst wird die Aufgabe durch ein Implantatmaterial gemäß Anspruch 1, das homogen verteilt eisendotierte Nanodiamanten in einer Matrix aus Magnesium oder einer Magnesiumlegierung umfasst. Gelöst wird die Aufgabe auch durch ein Verfahren zur Herstellung eines Implantatmaterials gemäß Anspruch 8, bei dem Magnesium oder eine Magnesiumlegierung aufgeschmolzen wird, zu der Schmelze eisendotierte Nanodiamanten gegeben werden, die mit eisendotierten Nanodiamanten versehene Schmelze aus Magnesium oder einer Magnesiumslegierung einer Ultraschallbehandlung unterzogen wird.

### DETAILLIERTE BESCHREIBUNG DER ERFIDUNG

Eisendotierte Nanodiamanten (Fe-NDs) sind beispielsweise aus B.-R. Lin et al. "Fe Doped Magnetic Nanodiamonds Made by Ion Implantation as Contrast Agent for MRI" Scientific Reports (2018) 8:7058 als Proteinmarker bekannt. Die eisendotierten Nanodiamanten werden bislang in der Forschung zum Sichtbarmachen von biologischen Zellprozessen eingesetzt. Eisendotierte Nanodiamanten sind für den Organismus unbedenklich und können als Kontrastmittel biologische Prozesse sichtbar machen.

Zur Herstellung der eisendotierten Nanodiamanten nehmen wir Bezug auf die *"Fe Doped Magnetic Nanodiamonds Made by Ion Implantation as Contrast Agent for MRI".* Nanodiamanten sind gut bekannt und können beispielsweise von Sigma-Aldrich käuflich erworben werden. Eisenionen lassen sich auf einfache Weise in diese Nanodiamanten implantieren. Dazu werden die Nanodiamanten bevorzugt in entmineralisiertem Wasser suspendiert und die Suspension anschließend auf eine Siliziumscheibe (Silicon Wafer) aufgetragen. Die Fe-Ionen können dann durch Sputtern in die Nanodiamanten implantiert werden. Bevorzugt wird dabei eine Energie von etwa 100-200 keV, wie etwa 150 keV, und eine Dosis von etwa 1 x 10¹⁵ Atomen/cm² bis 1 x 10¹⁶ Atomen/cm², wie etwa 5 x 10¹⁵ Atomen/cm² angewendet.

Das erfindungsgemäße Implantatmaterial aus Magnesium oder einer Magnesiumlegierung, das homogen verteilt eisendotierte Nanodiamanten enthält, kann mittels Einbringen der eisendotierten Nanodiamanten in eine Schmelze des Implantatmaterials hergestellt werden. Danach kann es stranggepresst werden oder mittels pulvermetallurgischer Verfahren wie der MIM-Technologie zu Implantat-körpern verarbeitet werden. Deren Lage im Körper des Patienten kann nun mit Hilfe der Margnetresonanztomographie (Magnetic Resonance Imaging, MRI) oder auf andere Weise nachgewiesen werden.

Mit der Resorption des Implantatmaterials im Körper des Patienten gelangen die eisendotierten Nanodiamanten in den Blutkreislauf. Die eisendotierten Nanodiamanten werden nach und nach aus dem Körper ausgeschieden. Der Abbau des Implantatmaterials kann ebenfalls mit Hilfe der Magnetresonanztomographie (Magnetic Resonance Imaging, MRI) oder auf andere Weise verfolgt werden.

Sofern eine Magnesiumlegierung als Matrixmaterial verwendet wird, werden bevorzugt Legierungselemente verwendet, die als nicht gesundheitsgefährdend gelten. Bevorzugt werden Magnesiumlegierungen mit Legierungselementen verwendet, die ausgewählt sind aus der Gruppe bestehend aus Lithium, Calcium, Kalium, Strontium, Barium, Scandium, Yttrium, Lanthan, Praseodym, Neodym, Samarium, Europium, Gadolinium, Dysprosium, Silizium, Kupfer, Zink, Gallium, Gold, Silber, Bismut, Eisen und Kombinationen davon. Bevorzugter werden Magnesiumlegierungen verwendet, wie sie in DE 10 2016 007 176 A1, oder DE 10 2016 119 227 A1 beschrieben sind.

Erfindungsgemäß wird das Implantatmaterial hergestellt, indem Magnesium oder eine Magnesiumlegierung aufgeschmolzen wird, zu der Schmelze Nanodiamanten gegeben werden und die mit Nanodiamanten versehene Schmelze aus Magnesium oder einer Magnesiumlegierung einer Ultraschallbehandlung unterzogen wird.

Ein derartiges Verfahren zur homogenen Verteilung von Nanopartikeln in einer Schmelze aus Magnesium oder einer Magnesiumlegierung wird in dem Artikel von H. Dieringa et al. "Ultrasound Assisted Casting of an AM60 Based Metal Matrix Nanocomposite, Its Properties, and Recyclability" in Metals 2017, 7, 338 beschrieben.

Bei einem bevorzugten Verfahren zur Herstellung des erfindungsgemäßen Implantatmaterials wird Magnesium oder eine Magnesiumlegierung bevorzugt in einem ersten Schritt in einer in einem Ofen befindlichen Kokille unter Schutzgas und unter Rühren aufgeschmolzen, in einem zweiten Schritt wird die Schmelze mit den eisendotieren Nanodiamanten versetzt und in einem dritten Schritt werden die in die Schmelze eingebrachten Nanodiamanten mittels einer Sonotrode dispergiert und deagglomeriert. Ein ähnliches Verfahren wird beispielsweise in H. Dieringa et al. "Ultrasound Assisted Casting of an AM60 Based Metal Matrix Nanocomposite, Its Properties, and Recyclability" in Metals 2017, 7, 338 beschrieben. Die Schmelze wird vorzugsweise mechanisch gerührt, vorzugsweise bei 150 bis 250 UpM. Anschließend werden die eisendotierten Nanodiamanten der Schmelze zugefügt. Nach Zugabe der eisendotierten Nanodiamanten wird die Schmelze mit Ultraschall behandelt. Dazu wird bevorzugt eine Sonotrode in die Schmelze eingebracht. Die Ultraschallbehandlung findet bevorzugt über eine Zeitspanne von 1 Min. bis 10 Min., bevorzugter 2 Min. bis 5 Min. statt.

Es ist ferner bevorzugt, dass die Kokille mit der Schmelze nach Herausnehmen des Rührers und der Sonotrode in ein Wasserbad getaucht wird. Somit erfolgt eine Erstarrung der Schmelze von "unten nach oben", wodurch eine Lunkerbildung vermieden wird.

Das erfindungsgemäße Implantatmaterial umfasst bevorzugt homogen verteilte eisendotierte Nanodiamanten in einer Matrix aus Magnesium oder einer Magnesiumlegierung in einer Menge von 0,01 bis 3 Gew.-%, bevorzugt 0,5 bis 1,5 Gew.-% auf Basis des Gewichts an Magnesium bzw. Magnesiumlegierung. Die Nanodiamanten haben bevorzugt eine Teilchengröße von 1 bis 20 nm, bevorzugt 3 bis 8 nm.

Das so hergestellte Implantatmaterial kann im Anschluss auf gewöhnliche Weise weiterverarbeitet werden. Beispielsweise kann das Implantatmaterial wieder aufgeschmolzen und anschließend in die gewünschte Form zur Bildung eines Implantatkörpers gegossen werden. Das Material kann auch stranggepresst werden, um aus dem Extrudat Implantate zu fertigen. Alternativ kann das Implantatmaterial zu Pulver weiterverarbeitet und mittels Metallspritzgiessen (Metal Injection Moulding, MIM) zu einem Implantatkörper weiterverarbeitet werden.

Das erfindungsgemäße Implantatmaterial kann auch mit Hilfe der MIM-Technologie zu einem metallischen Implantatkörper verarbeitet werden. Mit Hilfe der MIM-Technologie lassen sich kleine, komplexe und präzise geformte Metallbauteile endformnah fertigen. Die MIM-Technologie gehört zu den sogenannten pulvermetallurgischen Verfahren, bei denen kein massiver Metallkörper, sondern feines Metallpulver als Ausgangsmaterial für das herzustellende Bauteil verwendet wird. MIM steht dabei für "Metal Injection Moulding" (Metallpulverspritzguss). Beim MIM-Verfahren wird das Metallpulver durch Zusatz von thermoplastischen Bindemitteln fließfähig gemacht und das fließfähige Gemisch in eine Spritzgussform eingebracht. Nach der Formgebung wird der Bindemittelanteil wieder entfernt und das Bauteil gesintert. Magnesiumbauteile können mithilfe der MIM-Technologie nach dem in M. Wolff et. al. "Magnesium powder injection moulding for biomedical application", Powder Metallurgy, 2014 (Vol. 57, No. 5), 331-340 beschriebenen Verfahren hergestellt werden.

Das Bindemittel sorgt bei Anwendung der MIM-Technologie für eine temporäre Verbindung während des Urformens bzw. der Formgebung und sichert die Stabilität des Bauteils bis zur endgültigen Kompaktierung des Metallpulvers durch Sintern. Ein Teil des Bindemittels wird in der Regel bereits vor der Sinterung, zum Beispiel Mithilfe eines Lösungsmittels entfernt (Lösemittelentbinderung). Der Rest des Bindemittels zersetzt sich bei der thermischen Entbinderung bei Temperaturen von etwa 300°C bis 500°C und entweicht gasförmig.

## Patentansprüche

1. Implantatmaterial, das homogen verteilt eisendotierte Nanodiamanten in einer Matrix aus Magnesium oder einer Magnesiumlegierung umfasst.

2. Implantatmaterial nach Anspruch 1, wobei die homogen verteilten eisendotierten Nanodiamanten in einer Menge von 0,01 bis 3 Gew.-% auf Basis des Gewichts des Magnesiums bzw. der Magnesiumlegierung in der Matrix vorliegen.

3. Implantatmaterial nach Anspruch 2, wobei die homogen verteilten eisendotierten Nanodiamanten in einer Menge von 0,5 bis 1,5 Gew.-% auf Basis des Gewichts des Magnesiums bzw. der Magnesiumlegierung in der Matrix vorliegen.

4. Implantatmaterial nach einem der vorgehenden Ansprüche, wobei die eisendotierten Nanodiamanten eine Teilchengröße von Teilchengröße von 1 bis 20 nm aufweisen.

5. Implantatmaterial nach Anspruch 4, wobei die eisendotierten Nanodiamanten eine Teilchengröße von Teilchengröße von 3 bis 8 nm aufweisen.

6. Verfahren zur Herstellung eines Implantatmaterials gemäß einem der vorgehenden Ansprüche, bei dem Magnesium oder eine Magnesiumlegierung aufgeschmolzen wird, zu der Schmelze eisendotierte Nanodiamanten gegeben werden und die mit Nanodiamanten versehene Schmelze aus Magnesium oder einer Magnesiumslegierung einer Ultraschallbehandlung unterzogen wird.

7. Verfahren nach Anspruch 6, bei dem das Magnesium oder die Magnesiumlegierung in einem ersten Schritt in einer in einem Ofen befindlichen Kokille unter Schutzgas und unter Rühren aufgeschmolzen wird, die Schmelze mechanisch gerührt wird, anschließend die eisendotierten Nanodiamanten der Schmelze zugefügt werden und nach Zugabe der eisendotierten Nanodiamanten wird die Schmelze mit Ultraschall behandelt wird.

8. Verfahren nach Anspruch 7, wobei die Ultraschallbehandlung mittels einer in die Schmelze eingebrachten Sonotrode erfolgt.

9. Verfahren nach einem der Ansprüche 6 bis 8, bei dem die Ultraschallbehandlung über eine Zeitspanne von 1 Min. bis 10 Min. stattfindet.

10. Verfahren nach Anspruch 9, bei dem die Ultraschallbehandlung über eine Zeitspanne von 2 Min. bis 5 Min. stattfindet.

11. Verfahren nach einem der Ansprüche 7 bis 10, bei dem die Kokille nach der Ultraschallbehandlung in ein Wasserbad überführt wird, wo die Schmelze erstarrt.

12. Verfahren nach einem der Ansprüche 6 bis 11, bei dem das Implantatmaterial wieder aufgeschmolzen und anschließend in die gewünschte Form gegossen werden, um ein metallisches Implantat zu liefern.

13. Verfahren nach einem der Ansprüche 6 bis 11, bei dem das Implantatmaterial extrudiert wird und das Extrudat als Grundstoff für die Fertigung eines Implantates dient.

14. Verfahren nach einem der Ansprüche 6 bis 11, bei dem das Implantatmaterial mittels MIM-Technologie in ein metallisches Implantat überführt wird.

## Claims

1. An implant material comprising homogeneously distributed Fe-doped nanodiamonds in a matrix composed of magnesium or a magnesium alloy.

2. The implant material of Claim 1, wherein the homogeneously distributed Fe-doped nanodiamonds are present in the matrix in an amount of 0.01% to 3% by weight, based on the weight of the magnesium or the magnesium alloy.

3. The implant material of Claim 2, wherein the homogeneously distributed Fe-doped nanodiamonds are present in the matrix in an amount of 0.5% to 1.5% by weight, based on the weight of the magnesium or the magnesium alloy.

4. The implant material of any of the preceding claims, wherein the Fe-doped nanodiamonds have a particle size of 1 to 20 nm.

5. The implant material of Claim 4, wherein the Fe-doped nanodiamonds have a particle size of 3 to 8 nm.

6. A method for producing an implant material according to any of the preceding claims, in which magnesium or a magnesium alloy is melted, Fe-doped nanodiamonds are added to the melt, and the melt composed of magnesium or a magnesium alloy that has been provided with nanodiamonds is subjected to an ultrasound treatment.

7. The method of Claim 6, in which the magnesium or the magnesium alloy is melted under a protective gas and with stirring in a permanent mould situated in an oven in a first step, the melt is mechanically stirred and the Fe-doped nanodiamonds are subsequently added to the melt, and the melt is treated with ultrasound after addition of the Fe-doped nanodiamonds.

8. The method of Claim 7, wherein the ultrasound treatment is effected by means of a sonotrode introduced into the melt.

9. The method of any of Claims 6 to 8, in which the ultrasound treatment takes place over a period of 1 min to 10 min.

10. The method of Claim 9, in which the ultrasound treatment takes place over a period of 2 min to 5 min.

11. The method of any of Claims 7 to 10, in which the permanent mould is transferred to a water bath, where the melt solidifies, after the ultrasound treatment.

12. The method of any of Claims 6 to 11, in which the implant material is re-melted and then cast into the desired mould in order to yield a metallic implant.

13. The method of any of Claims 6 to 11, in which the implant material is extruded and the extrudate serves as base material for the manufacture of an implant.

14. The method of any of Claims 6 to 11, in which the implant material is converted into a metallic implant by means of MIM technology.

## Revendications

1. Matériau d'implant qui comprend des nanodiamants dopés au fer, répartis de manière homogène au sein d'une matrice en magnésium ou en un alliage de magnésium.

2. Matériau d'implant conforme à la revendication 1, dans lequel les nanodiamants dopés au fer et répartis de manière homogène se trouvent dans la matrice en une proportion de 0,01 à 3 %, en poids rapporté au poids du magnésium ou de l'alliage de magnésium.

3. Matériau d'implant conforme à la revendication 2, dans lequel les nanodiamants dopés au fer et répartis de manière homogène se trouvent dans la matrice en une proportion de 0,5 à 1,5 %, en poids rapporté au poids du magnésium ou de l'alliage de magnésium.

4. Matériau d'implant conforme à l'une des revendications précédentes, dans lequel les nanodiamants dopés au fer présentent une taille de particules qui vaut de 1 à 20 nm.

5. Matériau d'implant conforme à la revendication 4, dans lequel les nanodiamants dopés au fer présentent une taille de particules qui vaut de 3 à 8 nm.

6. Procédé de fabrication d'un matériau d'implant conforme à l'une des revendications précédentes, dans lequel on fait fondre du magnésium ou un alliage de magnésium, on ajoute à la masse fondue des nanodiamants dopés au fer, et l'on soumet cette masse fondue en magnésium ou en alliage de magnésium, pourvue de nanodiamants, à un traitement par ultra-sons.

7. Procédé conforme à la revendication 6, dans lequel, dans une première étape, on fait fondre le magnésium ou l'alliage de magnésium dans une lingotière placée dans un four, sous atmosphère de gaz protecteur et sous agitation, on agite mécaniquement la masse fondue, puis on ajoute à cette masse fondue les nanodiamants dopés au fer, et après l'addition des nanodiamants dopés au fer, on traite la masse fondue aux ultrasons.

8. Procédé conforme à la revendication 7, dans lequel on réalise le traitement aux ultrasons au moyen d'une sonotrode introduite dans la masse fondue.

9. Procédé conforme à l'une des revendications 6 à 8, dans lequel le traitement aux ultrasons se poursuit durant un laps de temps de 1 minute à 10 minutes.

10. Procédé conforme à la revendication 9, dans lequel le traitement aux ultrasons se poursuit durant un laps de temps de 2 minutes à 5 minutes.

11. Procédé conforme à l'une des revendications 7 à 10, dans lequel, après le traitement aux ultrasons, on transfère la lingotière dans un bain d'eau où la masse fondue se solidifie.

12. Procédé conforme à l'une des revendications 6 à 11, dans lequel on fait de nouveau fondre le matériau d'implant et on le coule ensuite dans un moule de la forme voulue, pour en faire un implant métallique.

13. Procédé conforme à l'une des revendications 6 à 11, dans lequel on extrude le matériau d'implant et le produit de cette extrusion sert de matériau de départ pour la fabrication d'un implant.

14. Procédé conforme à l'une des revendications 6 à 11, dans lequel on convertit le matériau d'implant en un implant métallique au moyen de la technique MIM.
